# EUROPÄISCHE PATENTANMELDUNG

(11) **EP 1 393 709 A1**
(43) Veröffentlichungstag der Anmeldung: **03.03.2004**
(21) Anmeldenummer: 03018168.9
(22) Anmeldetag: 07.08.2003
(51) Int. Cl.: A61K 7/135, A61K 7/06

(54) **Blondiermittel**

(30) Priorität: 20.08.2002 DE 10238970
(71) Anmelder: Henkel Kommanditgesellschaft auf Aktien, 40589 Düsseldorf (DE)
(72) Erfinder: Höffkes, Horst, Dr., 40595 Düsseldorf (DE)

(57) **Zusammenfassung**

Die Erfindung betrifft Mittel zum Blondieren menschlicher Haare auf Basis mindestens einer festen Peroxoverbindung und mindestens eines festen Alkaliträgers, die ein wasserlösliches Tensid auf Silikonbasis enthalten.

Die Mittel weisen ein sehr gutes Staubverhalten und ein exzellentes Mischungsverhalten auf.

## Beschreibung

Die Erfindung betrifft ein Blondiermittel für menschliche Haare, das ein wasserlösliches Tensid auf Silikonbasis enthält.

Die Veränderung von Form und Farbe der Haare stellt einen wichtigen Bereich der modernen Kosmetik dar. Dadurch kann das Erscheinungsbild der Haare sowohl aktuellen Modeströmungen als auch den individuellen Wünschen der einzelnen Person angepasst werden. So können beispielsweise Dauerwell- und andere die Haarform verändernde Verfahren sowie Färbe- und insbesondere Blondierverfahren für diese Zwecke angewendet werden. Die Grundlagen der Blondierverfahren sind dem Fachmann bekannt und können in einschlägigen Monographien, z.B. von Kh. Schrader, Grundlagen und Rezepturen der Kosmetika, 2. Auflage, 1989, Dr. Alfred Hüthig Verlag, Heidelberg, oder W. Umbach (Hrg.), Kosmetik, 2. Auflage, 1995, Georg. Thieme Verlag, Stuttgart, New York, nachgelesen werden.

Üblicherweise werden feste oder pastenförmige Zubereitungen mit festen Oxidationsmitteln unmittelbar vor der Anwendung mit einer verdünnten Wasserstoffperoxidlösung vermischt. Diese Mischung wird dann auf das Haar aufgebracht und nach einer bestimmten Einwirkzeit wieder ausgespült.

Die genannten Zubereitungen, die vor der Anwendung mit einer Wasserstoffperoxidlösung vermischt werden, werden im weiteren als "Blondiermittel" bezeichnet. Alle aufgeführten Angaben beziehen sich, soweit nicht anders ausgeführt, ausschließlich auf diese Zubereitungen.

Weder pastenförmige noch die pulverförmige Blondiermittel, die heute auf dem Markt sind, können als optimal angesehen werden. Während die Blondierwirkung auf dem Haar als befriedigend bezeichnet werden kann, bestehen doch noch eine Reihe von Nachteilen und Problemen sowohl bei Herstellung, als auch bei der Handhabung dieser Mittel. Bei pastenförmigen Mitteln, die aus Stabilitätsgründen hochviskos eingestellt werden müssen, können insbesondere die Dosierung und das Mischungsverhalten in der Wasserstoffperoxidlösung noch nicht befriedigen. Bei pulverförmigen Mitteln stehen das Staubverhalten, sowohl bei der Konfektionierung als auch bei der Anwendung, sowie ebenfalls das Mischungsverhalten bei der Anwendung, im Mittelpunkt der Verbesserungsbemühungen.

In der EP-B1-0 560 088 wurde beispielsweise vorgeschlagen, das Staubverhalten von Blondierpulvern durch Zugabe von Ölen oder flüssigen Wachsen zu verbessern. Diese Verbesserungen sollen durch Zugabe geringer Mengen, d.h. bis zu 2,5 Gew.-%, an nichtionischen Tensiden noch gesteigert werden.

In der EP-B1-0 672 408 wurden Blondierpulver mit 10 bis 27 Gew.-% eines wasserfreien Polymers (Polypropylenglycol) und in der DE 93 09 445 U1 mit einem Wachs versetzt, um das Staubverhalten zu verbessern

Die Anmeldungen DE 196 00 705 A1 und die DE 196 00 216 A1 haben ebenfalls Blondierpulver zum Gegenstand, die zur Verbesserung des Staubverhaltens Kohlenwasserstoffe oder spezielle Esterverbindungen enthalten.

Die Kombination einer festen Peroxoverbindung und einem nichtionischen Tensid ist aus der WO 97/23196 A1 bekannt. Insbesondere bevorzugt war dabei die Verwendung alkoxylierter Fettalkohole und Fettsäuren sowie alkoxylierter Sorbitan- und Polyglycerinester. Es wurde nun überraschenderweise gefunden, dass Blondierpulver mit vergleichbarem Staubverhalten aber signifikant verbessertem Mischungsverhalten erhalten werden, wenn sie mindestens ein wasserlösliches Tensid auf Silikonbasis enthalten.

Gegenstand der vorliegenden Erfindung sind daher Mittel zum Blondieren menschlicher Haare auf Basis mindestens einer festen Peroxoverbindung und mindestens eines festen Alkaliträgers, die ein wasserlösliches Tensid auf Silikonbasis enthalten.

Besonders bevorzugt sind erfindungsgemäß Blondiermittel, die in Pulverform vorliegen.

Die erfindungsgemäßen Blondiermittel enthalten als erste zwingende Komponente eine feste Peroxoverbindung. Die Auswahl dieser Peroxoverbindung unterliegt prinzipiell keinen Beschränkungen; übliche, dem Fachmann bekannte Peroxoverbindungen sind beispielsweise Ammoniumperoxidisulfat, Kaliumperoxidisulfat, Natriumperoxidisulfat, Ammoniumpersulfat, Kaliumpersulfat, Natriumpersulfat, Kaliumperoxidiphosphat, Percarbonate wie Magnesiumpercarbonat, Peroxide wie Bariumperoxid sowie Perborate, Harnstoffperoxid und Melaminperoxid. Unter diesen Peroxoverbindungen, die auch in Kombination eingesetzt werden können, sind erfindungsgemäß die anorganischen Verbindungen bevorzugt. Besonders bevorzugt sind die Peroxidisulfate, insbesondere Kombinationen aus mindestens zwei Peroxidisulfaten.

Die Peroxoverbindungen sind in den erfindungsgemäßen Blondiermitteln bevorzugt in Mengen von 20-80 Gew.-%, insbesondere in Mengen von 40-70 Gew.-% enthalten.

Als weitere zwingende Komponente enthalten die erfindungsgemäßen Blondiermittel ein Alkalisierungsmittel, das zur Einstellung des alkalischen pH-Wertes der Anwendungsmischung dient. Erfindungsgemäß können die dem Fachmann ebenfalls für Blondiermittel bekannten, üblichen Alkalisierungsmittel wie Ammonium-, Alkalimetall- und Erdalkalimetallhydroxyde, -carbonate, -hydrogencarbonate, -hydroxycarbonate, -silikate, insbesondere -metasilikate, sowie Alkaliphosphate verwendet werden. In einer bevorzugten Ausführungsform enthalten die erfindungsgemäßen Blondierpulver mindestens zwei unterschiedliche Alkalisierungsmittel. Dabei können Mischungen beispielsweise aus einem Metasilikat und einem Hydroxycarbonat bevorzugt sein.

Die erfindungsgemäßen Blondiermittel enthalten Alkalisierungsmittel bevorzugt in Mengen von 10-30 Gew.-%, insbesondere 15-25 Gew.-%.

Erfindungswesentlich ist auch die dritte zwingende Komponente der Blondierpulver, das wasserlösliche Tensid auf Silikonbasis. Dabei handelt es sich bevorzugt um solche wasserlöslichen Tenside auf Silikonbasis, die nichtionogener Natur sind.

Erfindungsgemäß bevorzugte wasserlösliche Tenside auf Silikonbasis sind weiterhin solche, die versprühbar sind.

Insbesondere bevorzugte wasserlösliche Tenside auf Silikonbasis sind ausgewählt aus der Gruppe der Dimethiconcopolyole die bevorzugt alkoxyliert, insbesondere polyethoxyliert oder polypropoxyliert sind.

Bevorzugt ist eine Einsatzmenge von 0,1 bis 20 Gew.-% des wasserlöslichen Tensids, insbesondere bevorzugt eine Einsatzmenge von 5 bis 15 Gew.-%.

Unter Dimethiconcopolyolen werden erfindungsgemäß Polyoxyalkylen-modifizierte Dimethylpolysiloxane der allgemeinen Formeln I oder II verstanden, 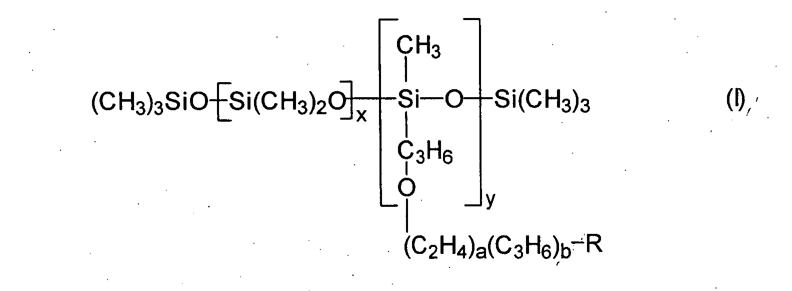 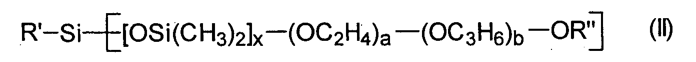 worin
- der Rest R für ein Wasserstoffatom, eine Alkylgruppe mit 1 bis 12 C-Atomen, eine Alkoxygruppe mit 1 bis 12 C-Atomen oder eine Hydroxylgruppe stehen kann,
- die Reste R' und R'' für Alkylgruppen mit 1 bis 12 C-Atomen stehen,
- x für eine ganze Zahl von 1 bis 100, bevorzugt von 20 bis 30 steht,
- Y für eine ganze Zahl von 1 bis 20, bevorzugt von 2 bis 10 steht und
- a und b stehen für ganze Zahlen von 0 bis 50, bevorzugt von 10 bis 30.

Verbindungen die unter die oben genannten Formeln fallen, werden in den folgenden Patentanmeldungen, auf die explizit Bezug genommen wird, offenbart: US-A-4,122,029; US-A-4,265,878; US-A-4,421,769 und GB-A-2,066,659.

Besonders bevorzugte Dimethiconcopolyole im Sinne der Erfindung sind beispielsweise die kommerziell unter dem Handelsnamen SILWET (Union Carbide Corporation) und DOW CORNING (Dow) vertriebenen Produkte.

Erfindungsgemäß besonders bevorzugte Dimethiconcopolyole sind Dow Corning 190 und Dow Corning 193.

In einer bevorzugten Ausführungsform der Erfindung wird das wasserlösliche Tensid auf Silikonbasis im Gemisch mit weiteren, nichtionogenen Tensiden verwendet.

Besonders geeignete nichtionogene oberflächenaktive Substanzen sind unter den Vertretern der im folgenden beschriebenen Klassen, wobei auch hier vorzugsweise Substanzen mit einem HLB-Wert von mindestens 5,0 verwendet werden. Dabei sind Substanzen, die kommerziell als Feststoffe oder Flüssigkeiten in reiner Form erhältlich sind, wegen der einfacheren Verarbeitbarkeit besonders bevorzugt.

Eine erste Klasse stellen alkoxylierte Fettalkohole mit 8 bis 22, insbesondere 10 bis 16, Kohlenstoffatomen in der Fettalkylgruppe und 1 bis 30, insbesondere 1 bis 15, Ethylenoxid- und/oder Propylenoxid-Einheiten dar. Bevorzugte Fettalkylgruppen sind beispielsweise Lauryl-, Myristyl-, Cetyl-, aber auch Stearyl-, Isostearyl- und Oleylgruppen. Wenngleich die alkoxylierten Fettalkohole sowohl Ethylenoxid als auch Propylenoxid-Einheiten enthalten können, so kann es doch bevorzugt sein, Substanzen auszuwählen, die nur einen Typ dieser Gruppen, insbesondere nur Ethylenoxid-Einheiten, enthalten. Besonders bevorzugte Verbindungen dieser Klasse sind beispielsweise Laurylalkohol mit 2 bis 4 Ethylenoxid-Einheiten, Oleyl- und Cetylalkohol mit jeweils 5 bis 10 Ethylenoxideinheiten und Cetyl- und Stearylalkohol sowie deren Mischungen mit 10 bis 30 Ethylenoxideinheiten. Ein bevorzugter ethoxylierter und propoxylierter Fettalkohol ist das Handelsprodukt Aethoxal®B (Henkel), ein Laurylalkohol mit jeweils 5 Ethylenoxid-und Propylenoxideinheiten.

Neben den üblichen alkoxylierten Fettalkoholen können auch sogenannte "endgruppenverschlossene" Verbindungen erfindungsgemäß eingesetzt werden. Bei diesen Verbindungen weist die Alkoxygruppe am Ende keine OH-Gruppe auf, sondern ist in Form eines Ethers, insbesondere eines C1-C4-Alkyl-Ethers, "verschlossen". Ein Beispiel für eine solche Verbindung ist das Handelsprodukt Dehypon®O 054, ein Octanol + 4,5 Ethylenoxid-butylether.

Weiterhin können, wie in den bevorzugten Verbindungen bereits erwähnt, anstelle spezieller Fettalkohole auch Mischungen eingesetzt werden, wie sie beispielsweise aus natürlichen Fetten und Ölen gewonnen werden. Eine bevorzugte Fettalkoholmischung wird durch die übliche Aufarbeitung von Kokosölen erhalten.

Schließlich können Verbindungen, die Anlagerungsprodukte von Ethylen- und/oder Propylenoxid an Fettalkohole darstellen, sowohl als Produkte mit einer "normalen" Homologenverteilung als auch in Form solcher Produkte mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder alkoholate als Katalysatoren verwendet werden. Die Verwendung/von Produkten mit eingeengter Homologenveteilung kann bevorzugt sein.

Eine zweite Klasse nichtionogener grenzflächenaktiver Substanzen stellen alkoxylierte Fettsäuren mit 8 bis 22, insbesondere 10 bis 16, Kohlenstoffatomen in der Fettsäuregruppe und 1 bis 30, insbesondere 1 bis 15, Ethylenoxid- und/oder Propylenoxid-Einheiten dar. Bevorzugte Fettsäuren sind beispielsweise Laurin-, Myristin-, Palmitin-, Stearin-, Isostearin- und Ölsäure. Wenngleich die alkoxylierten Fettsäuren sowohl Ethylenoxid als auch Propylenoxid-Einheiten enthalten können, so kann es doch bevorzugt sein, Substanzen auszuwählen, die nur einen Typ dieser Gruppen, insbesondere nur Ethylenoxid-Einheiten, enthalten. Besonders bevorzugte Verbindungen dieser Klasse sind beispielsweise Diethylenglykolmonostearat und -laurat sowie Tetraethylenglykolmonolaurat.

Eine dritte Klasse von geeigneten nichtionogenen grenzflächenaktiven Substanzen sind alkoxylierte, bevorzugt propoxylierte und insbesondere ethoxylierte, Mono-, Di- und Triglyceride. Beispiele für bevorzugte Verbindungen sind Glycerinmonolaurat + 20 Ethylenoxid und Glycerinmonostearat + 20 Ethylenoxid.

Eine weitere Klasse von geeigneten nichtionogenen grenzflächenaktiven Substanzen sind Polyglycerinester und alkoxylierte Polyglycerinester. Bevorzugte Verbindungen dieser Klasse sind beispielsweise Poly (3)glycerindiisostearat (Handelsprodukt: Lameform®TGI (Henkel)) und Poly(2)glycerin-polyhydroxystearat (Handelsprodukt: Dehymuls®PGPH (Henkel)).
Eine weitere Klasse von geeigneten nichtionogenen grenzflächenaktiven Substanzen sind Sorbitan-Fettsäureester und alkoxylierte Sorbitan-Fettsäureester wie beispielsweise Sorbitanmonolaurat und Sorbitanmonolaurat + 20 Ethylenoxid (EO).

Eine weitere Klasse bevorzugter nichtionogener grenzflächenaktiver Verbindungen sind schließlich Alkylphenole und Alkylphenolalkoxylate mit 6 bis 21, insbesondere 6 bis 15, Kohlenstoffatomen in der Alkylkette und 0 bis 30 Ethylenoxid- und/oder Propylenoxid-Einheiten. Bevorzugte Vertreter dieser Klasse sind beispielsweise Nonylphenol + 4 EO, Nonylphenol + 9 EO, Octylphenol + 3 EO und Octylphenol + 8 EO.

Besonders bevorzugte Klassen an nichtionogenen grenzflächenaktiven Stoffen stellen die alkoxylierten Fettalkohole, die alkoxylierten Fettsäuren sowie die Alkylphenole und Alkylphenolalkoxylate dar.

Als besonders vorteilhaft haben sich erfindungsgemäße Mittel erwiesen, die eine weitere nichtionogene grenzflächenaktive Substanzen in Mengen von 5 bis 15 Gew.-% enthalten.

Weiterhin können die erfindungsgemäßen Blondiermittel alle in solchen Zubereitungen bekannten Wirk-, Zusatz- und Hilfsstoffe enthalten. In vielen Fällen enthalten die Färbemittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, kationischen oder nichtionischen Tensiden auszuwählen. Anionische Tenside können dabei ganz besonders bevorzugt sein.

Bevorzugte anionische Tenside sind Alkylsulfate, Ethercarbonsäuren mit 10 bis 18 C-Atomen in der Alkylgruppe und bis zu 12 Glykolethergruppen im Molekül sowie insbesondere Salze von gesättigten und speziell ungesättigten C8-C22-Carbonsäuren wie Ölsäure, Stearinsäure, Isostearinsäure und Palmitinsäure.

Diese anionischen Tenside sollten bevorzugt in fester, insbesondere Pulverform vorliegen. Ganz besonders bevorzugt sind dabei bei Raumtemperatur feste Seifen, insbesondere Natriumstearat. Diese liegen bevorzugt in Mengen von 5 bis 20 Gew.-%, insbesondere 10 bis 15 Gew.-.%, vor.

Als nichtionische Tenside eignen sich insbesondere C8-C22-Alkylmono- und oligoglycoside und deren ethoxylierte Analoga. Insbesondere die nichtethoxylierten Verbindungen, die zudem in Pulverform kommerziell erhältlich sind, haben sich als besonders geeignet erwiesen.

Beispiele für die in den erfindungsgemäßen Haarbehandlungsmitteln verwendbaren kationischen Tenside sind insbesondere quartäre Ammoniumverbindungen. Bevorzugt sind Ammoniumhalogenide wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Tenside stellen die quaternisierten Proteinhydrolysate dar.

Alkylamidoamine, insbesondere Fettsäureamidoamine wie das unter der Bezeichnung Tego Amid®S 18 erhältliche Stearylamidopropyldimethylamin, zeichnen sich neben einer guten konditionierenden Wirkung speziell durch ihre gute biologische Abbaubarkeit aus.

Ebenfalls sehr gut biologisch abbaubar sind quaternäre Esterverbindungen, sogenannte "Esterquats", wie das unter der Bezeichnung Dehyquart®F 75 in Abmischung mit Cetearylalkohle erhältliche Distearoylethylhydroxyethylammoniummethosulfat.

Bei den eingesetzten Tensid-Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so dass man Substanzgemische mit unterschiedlichen, vom jeweiligen Rohstoff abhängigen Alkylkettenlängen erhält.

Weitere Wirk-, Hilfs- und Zusatzstoffe sind beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- kationische Polymere wie quaternisierte Celluloseether und andere, als Feststoff stabile bzw. im Handel erhältliche Verbindungen, zwitterionische und amphotere Polymere, die als Feststoffe stabil bzw. bevorzugt als Handelsprodukte erhältlich sind,
- anionische Polymere wie beispielsweise Polyacrylsäuren, vernetzte Polyacrylsäuren und Vinylacetat/Crotonsäure-Copolymere, sofern diese als Feststoffe stabil bzw. bevorzugt im Handel erhältlich sind,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z. B. Polyvinylalkohol,
- Strukturanten wie Glucose, Maleinsäure und Milchsäure,
- haarkonditionierende Verbindungen wie Phospholipide, beispielsweise Sojalecithin, Ei-Lecitin und Kephaline, sowie Silikonöle
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Farbstoffe zum Einfärben der Zubereitungen,
- Wirkstoffe wie Panthenol, Pantothensäure, Allantoin, Pyrrolidoncarbonsäuren und deren Salze,
- Cholesterin,
- Fette und Wachse wie Walrat, Bienenwachs, Montanwachs, Paraffine, Fettalkohole und Fettsäureester,
- Fettsäurealkanolamide,
- Komplexbildner wie EDTA, NTA und Phosphonsäuren,
- Quell- und Penetrationsstoffe wie Carbonate, Hydrogencarbonate, Guanidine, Harnstoffe sowie primäre, sekundäre und tertiäre Phosphate,

Die Auswahl dieser weiteren Stoffe wird der Fachmann gemäß der gewünschten Eigenschaften der Mittel treffen.

Da sich gezeigt hat, dass auf die Anwesenheit insbesondere von größeren Mengen an Ölen und flüssigen Wachsen, wie beispielsweise Paraffinöl, verzichtet werden kann, können erfindungsgemäße Mittel, insbesondere wenn das Haar nicht übermäßig beschwert werden soll, in einer bevorzugten Ausführungsform ohne diese Öle und flüssigen Wachse konfektioniert werden. Dabei ist klarzustellen, dass der Begriff Öle die bekannten fetten und synthetischen Öle, nicht aber Parfümöle umfasst, die selbstverständlich in geringen Mengen als Duftstoffe eingesetzt werden können.

Die Herstellung der erfindungsgemäßen Blondiermittel kann nach den üblichen, dem Fachmann bekannten Verfahren erfolgen.

Ein bevorzugtes Verfahren besteht darin, die anorganischen, als Feststoff vorliegenden Komponenten, gegebenenfalls nach Mischung (z. B. in einem Drais-Mischer) vorzulegen und mit dem grenzflächenaktiven Mittel zu besprühen. Dies erfolgt bevorzugt bei Raumtemperatur, d. h. bei Temperaturen unterhalb von ca. 30 °C; lediglich wenn die gewählten staubbindenden Komponenten bei diesen Temperaturen nicht als Flüssigkeit vorliegen, wird man erhöhte Temperaturen anwenden.

Ein weiteres Herstellungsverfahren für die erfindungsgemäßen Blondiermittel ist das Mischen aller Komponenten und die anschließende Behandlung, bevorzugt bei erhöhten Temperaturen, im Wirbelbett.

Schließlich ist es auch möglich, die erfindungsgemäßen Blondiermittel durch Vermahlen aller Komponenten in einer Kugelmühle, einer Ringwalzenmühle oder insbesondere einer Spindelmühle herzustellen.

Für die Anwendung der erfindungsgemäßen Mittel auf dem Haar werden die pulverförmigen Blondiermittel unmittelbar vor dem Auftragen mit einer Wasserstoffperoxid-Lösung vermischt. Die Konzentration dieser Wasserstoffperoxid-Lösung wird einerseits von den gesetzlichen Vorgaben und andererseits von dem gewünschten Effekt bestimmt; in der Regel werden 6- bis 12-prozentige Lösungen in Wasser verwendet. Die Mengenverhältnisse von Blondierpulver/und Wasserstoffperoxid-Lösung liegen dabei üblicherweise im Bereich 1:1 bis 1:2, wobei ein Überschuss an Wasserstoffperoxid-Lösung insbesondere dann gewählt wird, wenn keine zu ausgeprägte Blondierwirkung erwünscht ist.

Die folgenden Beispiele sollen den Erfindungsgegenstand verdeutlichen, ohne ihn darauf zu beschränken.

### Beispiele

| **Rohstoff** | **Rez.-Nr./Gew.-%** | | | |
|---|---|---|---|---|
| | **1** | **2** | **3** | **4** |
| Ammoniumperoxidisulfat | 19.0 | 19.0 | | - |
| Kaliumperoxidisulfat | 19.0 | - | 47.0 | - |
| Natriumperoxidisulfat | 16.0 | 35.0 | - | 44.0 |
| Magnesiumhydroxycarbonat | 3.0 | 3.0 | 3.0 | 3.0 |
| Natriumchlorid | - | - | 5.0 | - |
| Kaliumsulfat | - | - | - | 5.0 |
| Ammoniumchlorid | - | - | 2.0 | - |
| Ammoniumsulfat | - | - | - | 5.0 |
| Natriumstearat | 11.0 | 11.0 | 11.0 | 11.0 |
| Pyrogenes Siliziumdioxid | 2.5 | 2.5 | 2.5 | 2.5 |
| Dinatriumdihydrogenethylendiaminteraacetat | 1.0 | 1.0 | 1.0 | 1.0 |
| DC 190 | 7.5 | 7.5 | - | - |
| DC 193 | - | - | 7.5 | 7.5 |
| Trinatriumphosphat | 2.0 | 2.0 | 2.0 | 2.0 |
| Merquat 280 | 1.0 | 1.0 | 1.0 | 1.0 |

Die Rezepturen wurden bis auf die Tenside DC 190 und DC 193 sorgfältig gemischt und anschließend mit den Tensiden unter fortwährendem Mischen versprüht.

Alle Mittel ergeben beim Vermischen mit einer 6%igen Entwicklerzubereitung (Marktprodukt Poly Brillance) im Verhältnis 1:1 innerhalb kurzer Zeit homogene Mischungen. Dunkelblonde Haarsträhnen (Fischbach & Miller, Code 6923) von ca. 0,5 g Gewicht wurden mit jeweils ca. 2 g Blondiermischung 45 Minuten lang blondiert und ergaben vergleichbare Aufhellungen um 2-3 Nuancen.

## Patentansprüche

1. Mittel zum Blondieren menschlicher Haare auf Basis mindestens einer festen Peroxoverbindung und mindestens eines festen Alkaliträgers, **dadurch gekennzeichnet, dass** es ein wasserlösliches Tensid auf Silikonbasis enthält.

2. Mittel nach Anspruch 1, **dadurch gekennzeichnet, dass** es als Pulver vorliegt.

3. Mittel nach einem der Ansprüche 1 oder 2, **dadurch gekennzeichnet, dass** es 0,1 - 20 Gew.-% des wasserlöslichen Tensids auf Silikonbasis enthält.

4. Mittel nach einem der Ansprüche 1 bis 3, **dadurch gekennzeichnet, dass** es 5 bis 15 Gew.-% des wasserlöslichen Tensids auf Silikonbasis enthält.

5. Mittel nach einem der Ansprüche 1 bis 4, **dadurch gekennzeichnet, dass** das wasserlösliche Tensid auf Silikonbasis nichtionogener Natur ist.

6. Mittel nach einem der Ansprüche 1 bis 5, **dadurch gekennzeichnet, dass** das wasserlösliche Tensid auf Silikonbasis versprühbar ist.

7. Mittel nach einem der Ansprüche 1 bis 6, **dadurch gekennzeichnet, dass** das wasserlösliche Tensid auf Silikonbasis ausgewählt ist aus den Dimethiconcopolyolen.

8. Mittel nach einem der Ansprüche 1 bis 7, **dadurch gekennzeichnet, dass** das nichtionogene wasserlösliche Tensid auf Silikonbasis polyethoxyliert und/oder polypropoxyliert ist.

9. Mittel nach einem der Ansprüche 1 bis 8, **dadurch gekennzeichnet, dass** es eine anorganische feste Peroxoverbindung enthält.

10. Mittel nach einem der Ansprüche 1 bis 9, **dadurch gekennzeichnet, dass** es bei Raumtemperatur feste Seifen, insbesondere Natriumstearat, in Mengen von 5 - 20 Gew.-%, insbesondere 10 - 15 Gew.-%, bezogen auf das gesamte Pulver, enthält.

11. Mittel nach einem der Ansprüche 1 bis 10, **dadurch gekennzeichnet, dass** es frei von Ölen und flüssigen Wachsen ist, wobei Parfümöle ausgenommen sind.
